# EUROPEAN PATENT APPLICATION

(11) **EP 1 484 707 A2**
(43) Date of publication of application: **08.12.2004**
(21) Application number: 04253198.8
(22) Date of filing: 28.05.2004
(51) Int. Cl.: G06F 19/00

(54) **Medical image system, and medical image processing method**

(30) Priority: 03.06.2003 JP 2003157872; 17.09.2003 JP 2003324680; 17.09.2003 JP 2003324722
(71) Applicant: Konica Minolta Medical & Graphic Inc., Tokyo 163-0512 (JP)
(72) Inventor: Komiya, Yuji, Konica Minolta, Hachioji-shi Tokyo 192-8505 (JP); Umeki, Mamoru, Konica Minolta, Hachioji-shi Tokyo 192-8505 (JP); Nakazawa, Masayuki, Konica Minolta, Hachioji-shi Tokyo 192-8505 (JP); Toda, Haruyki, Konica Minolta, Hachioji-shi Tokyo 192-8505 (JP)
(74) Representative: Rees, Alexander Ellison

(57) **Abstract**

A medical image system including: an image data input section for inputting image data including medical image data of a radiographed object and supplementary information; a plurality of outputting sections for outputting a medical image on the basis of the medical image data; a setting section for setting a setting density range or a setting brightness range of the medical image to be outputted on the basis of the inputted supplementary information; an information obtaining section for obtaining density information including a output density range or brightness information including a output brightness range which each of the outputting sections can output; a selecting section for selecting at least one of the outputting sections which can output the medical image data; and a transmitting section for transmitting the medical image data with the supplementary information to the selected outputting sections.

## Description

### FIELD OF THE INVENTION

The present invention relates to a medical image system for outputting a medical image by converting the image signal inputted from a plurality of types of image signal sources into the image signal corresponding to the image signal source.

### BACKGROUND OF THE INVENTION

In the prior art, various methods have come to be devised to capture medical radiographic image information, without using a radiographic film consisting of silver halide photosensitive material. For example, there has been widespread use of Computed Radiography apparatus (hereinafter abbreviated as "CR") that utilizes the imaging plate mainly consisting of a stimulable phosphor to pick up photostimulated luminescence using excitation light after storing the radiographic image once, and applies photoelectric conversion to this light, thereby acquiring image signals. In recent years, a proposal has been made of a Flat Panel Detector (hereinafter abbreviated as "FPD") for reading radiographic image information by a combination of a radiographic phosphor, a radiographic conductor and a two-dimensional semiconducting detector such as a TFT switching device. Further, to meet particular requirements of each part of the body part to be radiographed and the purpose of radiographing, a computed tomography apparatus (hereinafter abbreviated as "CT"), a magnetic resonance imaging apparatus (hereinafter abbreviated as "MRI") and a mammographic apparatus (these apparatuses will be collectively called "medical image generation apparatus" hereinafter) are also employed.

These medical images are often diagnosed by the method of observing the hard copy where image information is recorded on a transmission recording medium and reflective recording medium. A medical image recording apparatus for recording medical image information on the recording medium includes the well known method of recording an image by laser exposure on a transmission recording medium using a silver halide recording material. This method permits a monochromatic image to be rendered in excellent gradations. At the same time, it provides an advanced level of diagnostic capacity by recording it on a transmission recording medium and observing under transmitted light. Various types of medical image recording apparatuses have been developed. A photosensitive heat development recording apparatus using a thermal head and heat mode laser and a photosensitive thermal development image recording apparatus using a photosensitive thermal development recording medium are also known, in addition to the method of using a silver halide recording medium requiring the prior art wet type processing.

In recent years, the aforementioned medical image generation apparatus and medical image recording apparatus are linked to a system (hereinafter referred to as "HIS" (Hospital Information System)) where the aforementioned medical image generation apparatus and medical image recording apparatus manage the information within a hospital and a system (hereinafter referred to as "RIS" (Radiology Information System)) for managing information in a radiology department, via the communications network such as LAN (Local Area Network). The radiographed medical image is transferred through the network, and is used in each apparatus. For example, the medical image generated in various types of medical image generation apparatuses is sent to the medical image recording apparatus, where the medical image is recorded on a recording medium and is outputted for use in diagnosis.

When medical images are recorded in these image recording apparatuses, a recording medium of a different density is used in conformity to each radiographed part of an object. For example, a recording medium having a higher density (e.g. Dmax = 4.0) than that of other general medical images is used in mammography. According to mammography, a higher possibility of early-stage cancer is present if there is a cluster of micro culcification. Accordingly, if a medical image is recorded on the recording medium of low density, contrast of the background of the recording medium and micro culcification image will be blurred, and diagnostic accuracy will be reduced in some cases. Thus, in the recording of a mammograph, the background image of the recording medium and subject image are outputted in high contrast. This requires the medical image to be recorded on a recording medium of high density.

In the mammography for diagnosing such a micro culcification image, a specifically high-resolution medical image is required for the very nature of diagnosis; hence the medical image must be recorded at a write pitch for smaller pixel size than that of other general medical images. For the reasons discussed above, in a system linked with a plurality of medical image generation apparatuses and composed of general medical images and mammographs, an operator manually selects a medical image recording apparatus having a write pitch in response to the characteristics of each medical image. In the selected medical image recording apparatus, the operator selects a recording medium having the density in conformity to the characteristics of each medical image, whereby the medical image is recorded, according to the prior art method.

The image recording apparatus for controlling a laser scanning means in response to the density of the recording medium includes an apparatus wherein the wedge patterns (test patterns) of a plurality of densities on the recording medium are exposed at every loading of a recording medium or a every predetermined interval, and the density of the obtained image is measured by a densitometer. According to the result of this measurement, the relationship between the input image signal and the amount of laser exposure is adjusted, and the variation of the photoconduction characteristics of a film and the fluctuation of development characteristics are automatically corrected, according to this disclosed technology (disclosed in Patent Document 1, for example).

### [Patent Document 1]

### Official Gazette of Japanese Patent Tokkaihei 6-233134

The aforementioned image recording apparatus (e.g. Patent Document 1) discloses a method of calibration for the variation of the film used, but fails to disclose the relationship between image information and output film when there are a plurality of films varying in the scope of density used.

When an image is displayed on a display monitor for diagnosis, the maximum brightness of the display monitor is often used in order to minimize the eye fatigue or glare of light. Further, there is a difference in the dynamic range, depending on the subject (part of an object). For example, a wider dynamic range than that of other part is used for the diagnosis of a breast, but the brightness is reduced; hence, the hardware capacity is not fully utilized in some cases.

### SUMMARY OF THE INVENTION

The object of the present invention is to provide a medical image system capable of capturing an image in response to the characteristics of a medical image, particularly to an medical image system for forming high quality medical image on a recording medium having the density corresponding. to the diagnosis of a subject's part to be examined, and a program for such a medical image system.

To achieve the aforementioned object, the present invention provides:
(1) A medical image system comprising:
   an image data input section for inputting an medical image accompanying the supplementary information including density information; and
   an image recording section for recording the medical image on a recording medium.

   This medical image system further consists of:
   a plurality of trays for loading recording media having different densities;
   a density information acquiring section for acquiring density information of the recording medium loaded into these trays;
   a transport section for transporting the recording medium loaded into a tray selected from a plurality of trays, to the image recording section; and
   a control section for determining the density of the recording medium based on the aforementioned supplementary information, and for selecting the tray to be loaded with the recording medium having the density corresponding to the determined density of the recording medium, based on the density information acquired from the density information acquiring section.
(2) The medical image system described in (1), wherein the image recording section allows at least two write pitches to be switched, and the control section sets the write pitch based on the supplementary information and switches the write pitch of the image recording section, thereby allowing the medical image to be recorded.
(3) The medical image system described in (2), capable of loading the recording medium having a different size for each of a plurality of the aforementioned trays.
(4) The medical image system described in any one of the aforementioned (1) through (3), wherein the image recording section records a medical image on a recording medium according to the photothermal silver halide method.
(5) A medical image system comprising:
   a plurality of medical image recording apparatuses;
   a plurality of medical image generation apparatus; and
   a control apparatus for acquiring an medical image accompanying the supplementary information including density information from the medical image generation apparatus and for sending this medical image to a predetermined medical image recording apparatus. This control apparatus further contains:
      a receiving section for receiving density information of the recording medium loaded in the tray of the medical image recording apparatus; and
      a transmitting section for determining the density of the recording medium based on the supplementary information of the medical image, and for transmitting the medical image accompanying the supplementary information to the medical image recording apparatus loading the recording medium corresponding to the determined density of the recording medium. The aforementioned medical image recording apparatus further includes:
         an image data input section for inputting a medical image accompanying the supplementary information including density information;
         a tray for loading the recording medium;
         a transmitting section for transmitting the density information of the recording medium loaded in the tray;
         an image recording medium for recording medical image on the recording medium; and
         a control section for selecting the tray to be loaded with the recording medium having the corresponding density based on the supplementary information of the medical image and the density information of the recording medium loaded in the tray.
(6) The medical image system described in (5), wherein the image recording section allows at least two write pitches to be switched, and the control section sets the write pitch based on the supplementary information and switches the write pitch of the image recording section, thereby allowing the medical image to be recorded.
(7) A medical image processing apparatus for receiving medical image information from other apparatus, applying image processing and outputting the result to one or more display apparatuses. This medical image processing apparatus includes:
   1) means for acquiring subject examination information from the received medical image and/or the supplementary information of that medical image;
   2) means for setting the drive level range (density range) on the display apparatus based on the subject examination information; and
   3) means for acquiring the maximum brightness information of the aforementioned one or more display apparatuses. This medical image processing apparatus is further characterized in that, based on the in the maximum brightness information, decision is made to select the display apparatus having the maximum brightness corresponding to the preset drive level (density range) out of the aforementioned one or more display apparatuses, and the output is displayed on that display apparatus.

The invention described in (1) determines the density of the recording medium for recording the medical image, based on the supplementary information accompanying the medical image; selects the tray to be loaded with the recording medium having the density corresponding to the determined density, transports the recording medium from the tray, and records the medical image on the recording medium having the density corresponding to the characteristics of the medical image. When the medical image is a mammograph, for example, this arrangement allows a medical image of high contrast to be formed on a high-density recording medium, and enhances diagnostic performances.

The invention described in (2) makes it possible to set the write pitch for recording a medical image, based on the supplementary information accompanying the medical image, and to record the medical image on the recording medium at the set write pitch. This arrangement provides high-definition medical image, for example, in the mammography requiring a high degree of sharpness, and allows a medical image to be formed in the general medical image, with a high priority given to processing efficiency.

The invention described in (3) makes it possible to use the recording media having different sizes, in addition to the recording media having different densities. This arrangement allows a medical image to be outputted, using a suitable recording medium corresponding to the type of the medical image.

The invention of (4) allows a medical image to be recorded on the recording medium without wet type processing.

The invention of (5) permits the controller to determine the density of the recording medium, based on the supplementary information, for example, when a recording medium of a different density is loaded for each of multiple image recording apparatuses. The medical image is sent to the medical image recording medium loaded with the recording medium corresponding to the determined density, whereby the medical image can be sent to the recording medium of a desired density with high accuracy and efficiency.

The invention of (6) makes it possible to set the write pitch for recording a medical image, based on the supplementary information accompanying the medical image, and allows the medical image to be recorded at the preset write pitch on the recording medium. This arrangement provides a medical image of high definition in the mammography requiring a high degree of sharpness, for example, and allows a medical image to be formed in the general medical image, with a high priority given to processing efficiency.

The invention of (7) is capable of setting the drive level range (density range) on the display apparatus based on the subject examination information acquired, and allows the output to be displayed on the display apparatus having the maximum brightness corresponding to the drive level (density range). This arrangement permits the image suitable for diagnosis of the subject to be displayed on the display apparatus.

### BRIEF DESCRIPTION OF THE DRAWINGS

Fig. 1 is a drawing representing the system configuration of a medical image system as an embodiment of the present invention;
Fig. 2 is a block diagram representing the functional configuration of an image recording apparatus 2 shown in Fig. 1;
Fig. 3 is a flowchart showing the process of medical image recording carried out by the image recording apparatus 2;
Fig. 4 is a system configuration of a medical image system 200 as another embodiment of the present invention;
Fig. 5 is a system configuration of a medical image system as another embodiment of the present invention;
Fig. 6 is a block diagram representing the configuration of a medical image processing apparatus 102;
Fig. 7 is a flowchart showing the first operation of a medical image processing apparatus of Figs. 5 and 6;
Fig. 8 is a flowchart showing the second operation of the medical image processing apparatus of Figs. 5 and 6;
Fig. 9 is a drawing schematically showing the setting of the maximum brightness of a monitor with respect to the image examination of the part of an object in the medical image processing apparatus 102 shown in Figs 5 and 6; and
Fig. 10 is a flowchart showing the operation of changing the maximum brightness setting of the monitor, based on the subject information in the medical image processing apparatus of Figs. 5 and 6.

### DETAILED DESCRIPTION OF THE PREFERRED EMBODIMENT

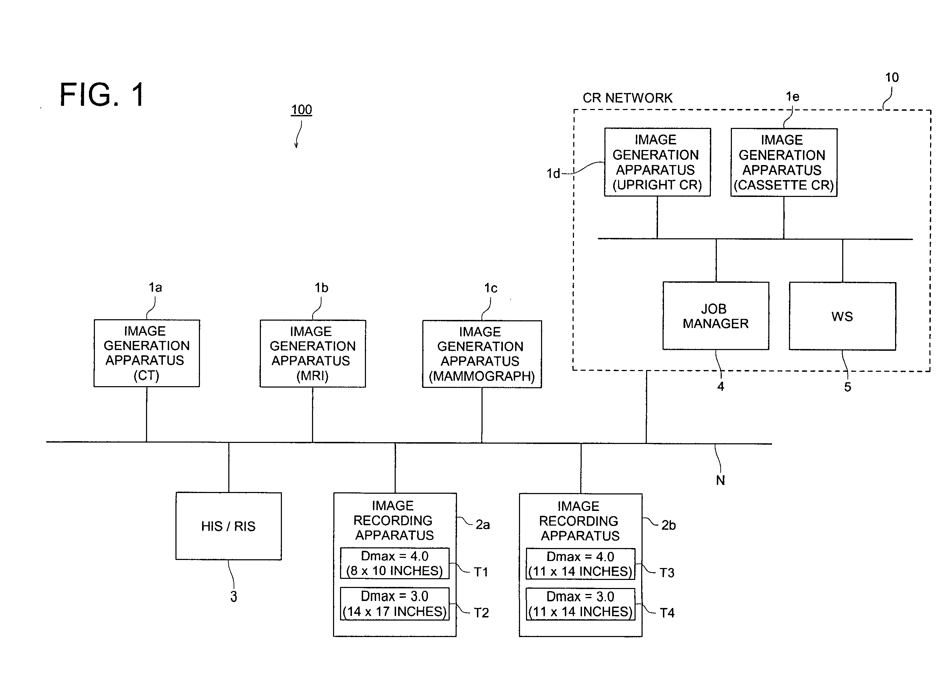

The following describes the embodiments of the present invention in details with reference to Figs. 1 through 10. It should be noted, however, that the scope of the invention is not restricted to the illustrated examples.

The following describes the configuration of the present embodiment:

Fig. 1 is a conceptual diagram representing the system configuration of a medical image system 100 in the present embodiment. As shown in Fig. 1, the medical image system 100 is linked with medical image generation apparatuses 1a through 1e, medical image recording apparatuses 2a and 2b, HIS/RIS3, JOB manger 4, WS (Work Station) 5 and others through the network so that data can be transferred among them. Further, medical image generation apparatuses 1d and 1e, JOB manager 4 and WS5 constitute the CR network 10.

LAN (Local Area Network), WAN (Wide Area Network), the Internet and various forms of lines can be applied as the network N. Radio communications and infrared communications can be used if authorized by a medical institution such as a hospital. Since important patient information is contained, it is preferred that the information to be transmitted and received be encrypted. Further, the DICOM (Digital Image and Communications in Medicine) standard is commonly used as a communications system in the hospital. DICOM MWM (Modality Worklist Management) and DICOM MPPS (Modality Performed Procedure Step) are used in the communications in the aforementioned medical image generation apparatuses 1a through 1e and medical image recording apparatuses 2a and 2b.

The medical image generation apparatuses (hereinafter referred to as "image generation apparatuses") 1a through 1e consists of a CR, FPD, CT, MRI, mammographic X-ray apparatus, etc., for example. It is used to radiograph a subject, applies digital conversion to the radiographed image, and generates a digital medical image. In the present embodiment, the following explanation uses the example when the image generation apparatus 1a is the CT, the image generation apparatus 1b the MRI, the image generation apparatus 1c the mammographic X-ray apparatus, the image generation apparatus 1d the upright CR, and the image generation apparatus 1e the cassette CR.

It is assumed that the image generation apparatuses 1a through 1e conform to the aforementioned DAICOM standard, and are provided with the supplementary information in conformity to the DICOM standard. When the image generation apparatuses 1a through 1e fails to conform to the DAICOM standard, the DICOM converter (not illustrated) is used, and DICOM supplementary information is entered. It is also possible to arrange such a configuration that supplementary information is generated by the Job Manager 4 or WS5 to be described later, and is attached to the medical image.

The medical image recording apparatuses (image recording apparatuses) 2a and 2b output the hard copy wherein the image data inputted from the image generation apparatuses 1a through 1e is reproduced as a visible image. They are composed of various types of dry printers using an X-ray laser, heat mode laser, thermal head, etc., for example.

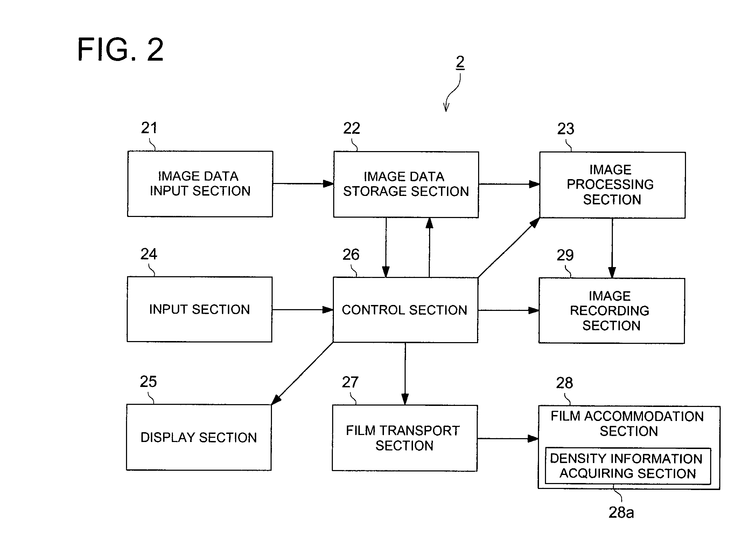

The following describes the image recording apparatuses 2a and 2b with reference to Fig. 2. The image recording apparatuses 2a and 2b each have the same configuration, hence, the same components are assigned with the same numerals, and the image recording apparatus 2a (shown as an image recording apparatus 2 in Fig. 2) will be used as an example for the following explanation. Fig. 2 is a block diagram representing the functional configuration of an image recording apparatus 2a. As shown in Fig. 2, the image recording apparatus 2a comprises an image data input section 21, image data storage section 22, image processing section 23, input section 24, display section 25, control section 26, film transport section 27, film accommodation section 28, image recording section 29, etc.

The image data input section 21 acquires the digitally converted medical image from the image generation apparatuses 1a through 1c through the network N. It is also possible to arrange such a configuration that the image data input section 21 reads medical image from various storage medium such as the CD-ROM (Compact Disk-Read Only Memory), floppy registered trademark), and storage of PACS system.

Alternatively, it is also possible to arrange such a configuration that the image data input section 21 is a laser digitalizer or the like composed separately from the image recording apparatus 2, and the film containing the medical image of a patient is scanned by a laser beam. The amount of transmitted light is measured and the measurement is subjected to analog-to-digital conversion, whereby the medical image is inputted into the image recording apparatus 2 as a digital image data.

Without being restricted to the aforementioned laser digitizer, the image data input section 21 can also be configured in such a way that the light detecting device such as CCD (charge coupled device) is used to scan the film with medical image recorded thereon by the beam, and the reflected light is subjected to photoelectric conversion by the CCD. Then the digital image data is inputted.

Further, instead of the configuration where the medical image recorded on the film is read, it is also possible to arrange such a configuration that the image data input section 21 can be connected to the radiographic apparatus where the medical image radiographed by a stimulable phosphor is subjected to digital conversion, and the medical image data is generated. From this radiographic apparatus, the digital image data is inputted into the CR system network. In this case, there is no need of using a film, and this will contribute to cost reduction.

Further, the image data input section 21 can also be configured in such a way that it can be linked with an FPD that captures a radiographic image and outputs it in the form of an electric signal. Then the digital image data can be inputted from this FPD. The FPD consists of two-dimensional arrangement of the radiation detecting device for generating electric charge in conformity to the intensity of the applied radiation, and the capacitor that accumulates the electric charge generated by this radiation detecting device, as disclosed in the Official Gazette of Japanese Patent Tokkaihei 6-342098.

As disclosed in the Official Gazette of Japanese Patent Tokkaihei 9-90048, it is also possible to arrange such a configuration that the image data input section 21 is provided with a light detector which is equipped, for each of pixels, with a light detecting device for detecting the intensity of fluorescent light such as a photodiode, CCD, CMOS (Complementary Metal Oxide Semiconductor) sensors. Radiation is absorbed by the phosphor layer such as X-ray intensifying screen so that fluorescent light is emitted, and the intensity of this fluorescent light is detected by the light detector. The result is subjected to photoelectric conversion and digital medical image data is inputted. It is also possible to use a combination of a radiation a scintillator for emitting visible light in response to application of radiation and a lens eye with an area sensor corresponding to each lens eye.

The medical image inputted from the image data input section 21 contains a header area. The header area is filled with information on the medical image according to the DICOM standard. This information consists of:
information on the radiographed patient such as the name, patient ID (for identification of each patient) and gender;
information on radiographing such as the part of an object and date of radiographing, and examination ID showing the type of examination related to the image (ID for identifying each examination); and
supplementary information such as an apparatus ID of the image generation apparatuses having generated the medical image (ID for identifying each apparatus), recording pixel size (read pitch), recording medium size and recording medium density information.

The image data storage section 22 consists of a magnetic, optical recording medium or semiconductor memory, and stores the medical image inputted by means of the image data input section 21. In this case, the data is compressed, as required. The data can be compressed in reversible or irreversible mode according to the known JPEG, DPCM or Weblet compression techniques. Irreversible compression is preferred because deterioration of image data does not occur as a result of compression.

The image processing section 23 applies various type of image processing to the medical image data inputted from the image data storage section 22, and outputs the result to the image recording section 29. These various type of image processing includes gradation processing for adjusting the image contrast, contrast correction, frequency enhancement for adjusting the sharpness of the image, dynamic range compression for keeping an image of wide dynamic range within easy-to-see range of density, without reducing the contrast on the details of a subject, and correction for same size (same size of a subject) outputting of the medical image having different read pixel size and recording image size.

The input section 24 consists of a keyboard, mouse and others, and generates the instruction signal corresponding to the inputted instruction. This signal is outputted to the control section 26. To put it more specifically, in the mode set by the control section 26, the recording medium size to be described later, recording image size and tray as an output destination are inputted, and are outputted to the control section 26.

The display section 25 is a display means consisting of a CRT (cathode ray tube), LCD (liquid crystal display) and plasma display, and outputs the display information inputted from the control section 26.

The control section 26 acquires the DICOM supplementary information accompanying the header of the medical image read by the image data storage section 22 and determines the preferable density of the recording medium for recording a medical image. To put it more specifically, it determines the preferred density of the recording medium for recording the medical image, based on the read pitch contained in the supplementary information and/or part information. To put it another way, in addition to the case where high-resolution reading is restricted to mammography, when high-resolution reading is applied to other than radiographing of a breast, the part information (radiographed part of an object) is also taken in account in determining the density used for outputting. In this case, the preferred approximate density of the recording medium is Dmax = 3.6 through 4.0 when the medical image is a mammographic image, and Dmax = 3.0 when the medical image is a general medical image (e.g. medical image generated by the CT, MRI, upright CR). In the present embodiment, it is assumed that the preferred density in the case of a mammographic image is Dmax = 4.0, and that for general medical image is Dmax = 3.0. Based on this assumption, the following description is given.

The supplementary information for determining the preferred density of the recording medium for recording the medical image includes the maximum density (Dmax) information at the time of recording, the type of the image generation apparatus (identification information), recording medium size, reading pixel size (read pitch) and the part of an object to be radiographed. For example, when the supplementary information contains the Dmax information at the time of recording, the density of the recording medium is determined based on this Dmax information.

When the supplementary information does not include the information on the type of the image generation apparatuses 1a through 1e, the image is regarded as a general medical image if the apparatus is a CT, MRI and upright CR. The image is regarded as a mammographic image if the apparatus is a mammographic apparatus. In this manner, density suitable for each image is determined. If the apparatus is a cassette CR, decision is made according to other information since the type of the medical image cannot be determined according to the type of the apparatus.

When the supplementary information includes the information on the size of the recording medium, the image regarded as a general medical image if the recording medium size is 14" x 17". It is regarded as a mammographic image if the size is 8" x 10". In this manner, density suitable for each image is determined. If the recording medium size is 11" x 14", decision is made according to other information since the type of the medical image cannot be determined according to the recording medium size. Further, various pieces of information contained in the supplementary information and combinations thereof are used to determine the type of the medical image.

In the mammography requiring a high-resolution outputting, the medical image is read at a read pitch for small pixel size. In the meantime, the other general medical images are read at a pitch coarser than that in the mammography, with consideration given to the processing efficiency, data capacity and the transmission load in the network. Thus, the control section 26 acquires the read pitch of the medical image contained in the supplementary information accompanying the medical image, and evaluates if the medical image is a mammographic image or general medical image, based on the read pitch, thereby determining the density of the recording medium suitable for each medical image. To put it more specifically, in the present embodiment, the read pitch of the mammographic image is 50 microns and that of the general medical image is 87.5 microns. Based on this example, the following describes the present embodiment:

The control section 26 obtains the density information of the film loaded in the trays T1 and T2. The control section 26, for example, outputs to the film transport section 27 a selection signal that selects a tray loaded with a film having a density of Dmax = 4.0 if the read pitch is for small pixel. Further, when the medical image is a general medical image, it outputs to the film transport section 27 a selection signal that selects a tray loaded with a film having a density of Dmax = 3.0.

The control section 26 compares the Dmax information contained in the supplementary information with the density information of the film loaded in each tray, and determines the tray that allows outputting. If the Dmax is not included in the supplementary information, evaluation is made, based on the supplementary information, to determine whether the medical image is a mammographic image or not. If it has determined that it is a mammographic image, the control section 26 outputs to the film transport section 27 the selection signal for selecting the tray loaded with a film of Dmax = 4.0. When evaluation has been made to determine that the medical image is a general medical image, the control section 26 outputs to the film transport section 27 the selection signal for selecting the tray loaded with a film of Dmax = 3.0.

When the control section 26 has determined, based on the read pitch or supplementary information, that the medical image is a mammographic image, it outputs the control signal for setting the write at high-resolution 25 microns, to the image recording section 29. When the control section 26 has determined that the medical image is a general image, it outputs the control signal for setting the write at 50 microns to the image recording section 29. When the general medical image is to be recorded on the recording medium, a high-resolution medical image can be formed if the write pitch is on the order of 50 microns. However, in the mammography requiring particularly high-resolution outputting, the write pitch is prefer to be set at a fine level of about 25 microns. It is also possible to set the write pitch for general medical image at 25 microns to form an image, but this will take more time in image processing and will reduce the entire performance. This is not preferred from the practical viewpoint.

The film transport section 27 is composed of a film pickup for picking up a film form the film accommodation section 28, a transfer roller and others. It selects a specified tray T1 or T2 in response to the selection signal sent from the control section 26, and transports the recording medium loaded in the selected tray, to the image recording section 29.

The film accommodation section 28 is equipped with trays T1 and T2. Trays T1 and t2 can be loaded with a plurality of film packages different in density (a mammographic film having a maximum density Dmax = 4.0 and a film for general medical image having a maximum density Dmax = 3.0). The film accommodation section 28 accommodates the film of the loaded film package. As shown in Fig. 1, the tray T1 accommodates an 8 x 10-inch sized recording medium having a density of Dmax = 4.0, whereas the tray T2 accommodates a 14 x 17-inch sized recording medium having a density of Dmax = 3.0. The number of the trays provided on the film accommodation section 28, and the density or size of the recording medium to be accommodated therein, can be determined as desired, without being restricted to the aforementioned values.

The recording medium stored in the trays 1 through 4 is made of a photosensitive development recording medium or photosensitive thermal development recording medium, for example. In this recording medium, a photocatalyst such as photosensitive silver halide forms a latent image when exposed to light, and the silver of the organic silver salt ionized by the action of a reducing agent migrates to bond with photosensitive silver halide, thereby creating crystalline silver and forming an image.

The film accommodation section 28 is equipped with a density information acquiring section 28a to get the density information of the loaded film. The density information acquiring section 28a has a barcode reader and reads the barcode of the film package loaded in the film accommodation section 28. It acquires film information including the density and type of the film obtained from the barcode, and outputs the film density information to the control section 26. It is also possible to arrange such a configuration that the density information acquiring section 28a has a densitometer to measure the density of the film developed at the time of calibration and to determine the maximum density, thereby getting density information of the loaded film.

The recording medium is exposed to a laser beam modulated in response to the medical image data inputted from the image processing section 23, whereby a latent image is formed. After thermal developing, the latent image becomes visible. Further, the image recording section 29 adjusts the diameter of the laser beam produced under the control of the control section 26, whereby the write pitch (pixel size) is switched between 50 microns (for general medical image) and 25 microns (for mammographic image), and the medical image is recorded.

The following describes the image recording apparatus 2b. The image recording apparatus 2b has the same configuration as that of the image recording apparatus 2a. Accordingly, the same numerals will be assigned to the same components, and illustration and detailed description will be omitted.

The film accommodation section 28 is equipped with trays T3 and T4. Trays T3 and T4 are loaded with the recording media having the same size but different densities. To put it more specifically, the tray T3 accommodates a 11 x 14-inch sized recording medium having a density of Dmax of 4.0, while the tray T4 accommodates a 11 x 14-inch sized recording medium having a density of Dmax of 3.0. That a mammographic image is to be outputted on the 11 x 14-inch sized recording medium means that a right/left breast image is outputted onto one recording medium so that right and left breasts are diagnosed at the same time.

The image recording section 29 adjusts the diameter of the laser beam produced under the control of the control section 26. When recorded on the recording medium (Dmax = 4.0) transported from the tray T3, the write pitch is switched to 25 microns (for mammographic image), and the medical image is recorded. When recorded on the recording medium (Dmax = 3.0) transported from the tray T4, the write pitch is switched to 50 microns (for general medical image), and the medical image is recorded.

The HIS/RIS3 is an information system configured in a hospital through the network, and is composed of a server for the management of the overall system, a terminal and a database. To put it more specifically, the HIS/RIS3 generates or accepts an order of radiographing a medical image and manages the examination data and radiographed medical image.

The Job Manager 4 manages the medical image among the image generation apparatuses 1a through 1e, image recording apparatuses 2 and database (not illustrated), based on the DICOM supplementary information. For example, when there is an input of the medical image generated by an image generation apparatus not corresponding to the DICOM standard, the Job Manager 4 generates the supplementary information corresponding to the DICOM standard and manages the medical image. When the medical image generated by the image generation apparatuses 1a through 1e is to be recorded on the recording medium, the Job Manager 4 selects the image recording apparatus 2a or 2b, based on the supplementary information, and outputs the medical image to the corresponding image recording apparatus, whereby the medical image is recorded.

The WS5 is a network server for configuring the CR network and administers the image generation apparatuses 1d and 1e, Job Manager 4 and others.

The following describes the operation of the present embodiment:

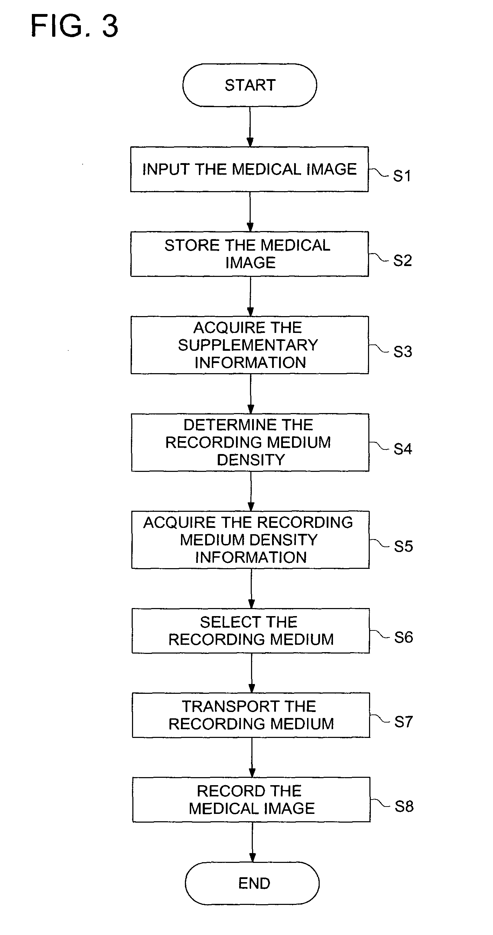

Fig. 3 is a flowchart showing the process of medical image recording carried out by the image recording apparatuses 2a and 2b. The following describes the operations of the image recording apparatuses 2a and 2b with reference to Fig. 3:

When a medical image is inputted from the image data input section 21 (Step S1), the medical image is recorded in the image data storage section 22. At the same time, the medical image is outputted to the image processing section 23 and control section 26 (Step S2). Then the supplementary information accompanying the medical image is acquired by the control section 26 (Step S3), and the density of the recording medium is determined based on the acquired supplementary information (Step S4).

Here the control section 26 of the image recording apparatus 2a determines the density suitable for the recording medium for recording the medical image, based on the Dmax information at the time of recording, the type of the apparatus and the part of an object to be radiographed, contained in the supplementary information. The control section 26 of the image recording apparatus 2b determines the density suitable for the recording medium for recording the medical image, based on the Dmax information at the time of recording, the type of the apparatus and the part of an object to be radiographed, contained in the supplementary information, since the loaded recording media have the same size.

When the density of the recording medium is determined according to the read pitch of the medical image contained in the acquired supplementary information, the control section 26 of the image recording apparatus 2a determines the density of the recording medium to be Dmax = 4.0, if the read pitch contained in the supplementary information is 50 microns. It determines the density of the recording medium to be Dmax = 3.0 if the read pitch contained in the supplementary information is 87.5 microns.

Based on the information inputted from the density information acquiring section 28a, the density information of the film loaded in each of the trays T1, T2 (or T3 and T4) is acquired (Step S5). Then the tray for supplying the recording medium is selected by the control section 26, based on the determined density of the recording medium and the density information of the film loaded on the tray T1 or T2 (tray T3 or T4), and the tray selection signal is outputted to the film transport section 27 (Step S6).

When the medical image is a mammographic image and the preferred density of Dmax = 4.0 is determined, the control section 26 selects the tray loaded with a recording medium having a density of Dmax = 4.0, based on the density information entered from the density information acquiring section 28a; namely the tray T1 (or tray T3), and outputs the selection signal. Further, when the medical image is a general medical image and the preferred density of Dmax = 3.0 is determined, the control section 26 selects the tray loaded with a recording medium having a density of Dmax = 3.0, based on the density information entered from the density information acquiring section 28a; namely the tray T2 (or tray T4), and outputs the selection signal.

According to the selection signal outputted from the control section 26, the recording medium loaded on the tray T1 or T2 (tray T3 or T4) is transported to the image recording section 29 by the film transport section 27 (Step 36).

Then the write pitch of the image recording section 29 is switched according to the types of the medical image or read pitch of the medical image, and the recording medium is exposed to the laser beam having modulated the medical image data, by the image recording section 29 (Step S8).

The write pitch is switched according to the control signal outputted from the control section 26. When the medical image is a mammographic image, the write pitch is set to 25 microns by the control section 26 since a high definition is required. If the medical image is a general image, the write pitch is set to 50 microns by the control section 26 in order to improve processing efficiency. If the read pitch is 50 microns, it is estimated that high definition is required and the rite pitch is set at 25 microns. If the read pitch is 87.5 microns, the write pitch is set at 50 microns.

As described above, in the present embodiment, the image recording apparatus 2 determines the density of the recording medium for recording the medical image, based on the supplementary information accompanying the medical image (including the read pitch at the time of photographing the medical image). It selects the tray loaded with the recording medium having the density corresponding to the type of the medical image, and transports the recording medium from that tray. It also switches the write pitch of the image recording section 29 over to the write pitch corresponding to the characteristics (including the read pitch) of the medical image, and records the medical image on the recording medium having the suited density.

Thus, in the image recording apparatus, connected to a plurality of image generation apparatuses 1a through 1e, where general medical image and mammographic image are inputted in fixed form, it is possible to determined the density of the recording medium for recording the medical image according to the supplementary information accompanying the medical image, and to select a tray for loading the recording medium having the density corresponding to the determined density. Then the recording medium is transported from that tray and the medical image is recorded in the recording medium corresponding to the characteristics of the medical image. This arrangement allows a sharp medical image to be formed, with the result that diagnostic performances are upgraded.

According to the supplementary information accompanying the medical image, the write pitch is switched and the medical image is recorded. Thus, in the mammography requiring a very sharp image, high-definition medical image can be provided. In the case of a general medical image, a higher priority can be given to the processing efficiency when forming a medical image.

Based on various pieces of information included in the supplementary information, the density of the recording medium and preferred write pitch can be determined. Even if the supplementary information is not completely standardized, requirements can be realized, and a highly versatile system can be provided.

The image recording apparatuses 2a and 2b are provided with a plurality of trays, which are loaded with recording media having different densities. The optimum tray is selected in conformity to the type of the medical image and the recording medium loaded in that tray is transported to the image recording section 29. Thus, when the medical image is outputted, this arrangement prevents a wrong tray from being selected by an operator, and the medical image from being recorded onto the recording medium having different density. This arrangement also eliminates wasteful use of a recording medium.

The film accommodation section 28 contains a density information acquiring section 28a, which acquires the density information of the film loaded on the tray T1 or T3. Thus, a tray loaded with the recording medium having a density corresponding to the medical image is selected and the medical image is transported from that tray. This arrangement prevents a tray having a wrong density from being recorded onto the recording medium, and eliminates wasteful use of a recording medium.

For example, even when a tray has been loaded with the recording medium of wrong density, the density information is outputted to the control section 26 by the density information acquiring section 28a. Based on this density information, the recording medium is transported. Because of this control, replacement of the loaded film does not require much time, and this provides an extra convenience.
Especially in the case of a film package that does not allow replacement of the film once the film package has been loaded on the tray, the entire film package may be waste if the tray is loaded with a recording medium of a different density. In the present embodiment, however, the film transport control is carried out in response to the density of the film actually loaded into the tray, and this arrangement eliminates the wasteful use of an expensive recording medium. This arrangement also provides an image recording apparatus of high versatility since the recording medium of any density can be loaded, without its density being fixed for each tray.

The description in the aforementioned embodiment is applicable to an example of the preferred image recording apparatus of the present invention, without being restricted thereto.

In the present embodiment, the image recording apparatus 2a has two trays, T1 and T2, for accommodating two types of recording media having different densities and sizes. Two types of write pitches are switched according to the type of the medical image, whereby the medical image is recorded. However, the present invention is not restricted to this description. It goes without saying that it is possible to change, as desired, the density and size of the recording medium, number of trays and type of the write pitches that can be switched. Further, the image recording apparatus 2b is equipped with two types of trays, T3 and T4, for loading the recording media of the same size and different densities, and two write pitches are switched to conform to the each type of the medical image, whereby the medical image is recorded, according to the aforementioned description. However, the present invention is not restricted to this arrangement. It goes without saying that it is possible to change, as desired, the density and size of the recording medium, number of trays and type of the write pitches that can be switched.

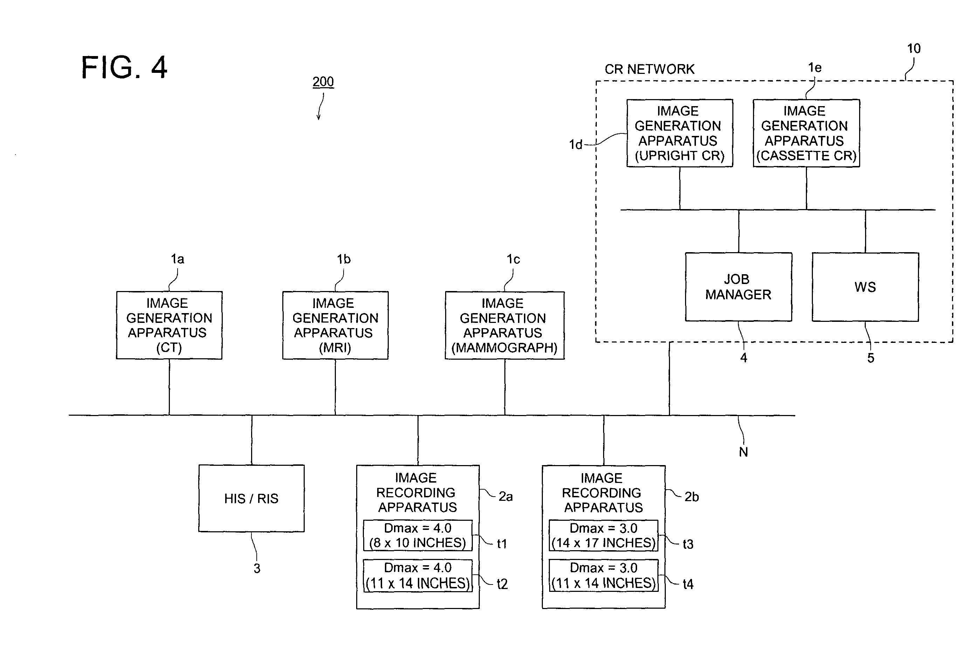

For example, in the medical image system 200 shown in Fig. 4, as another embodiment, it is possible to arrange the image recording apparatuses 2a and 2b in such a configuration that the recording media of the same density and difference sizes can be accommodated; i.e. a 8 x 10-inch sized recording medium having a density of Dmax = 4.0 is accommodated in the tray T1; a 11 x 14-inch sized recording medium having a density of Dmax = 4.0 in the tray T2; a 14 x 17-inch sized recording medium having a density of Dmax = 3.0 in the tray T3; and a 11 x 14-inch sized recording medium having a density of Dmax = 3.0 in the tray T4.

In this case, the medical image outputted from the image generation apparatuses 1a through 1e is obtained by the Job Manager 4. The Job Manager 4 determines the density of the recording medium for recording the medical image, based on the information on the density of the recording medium and type of the medical image, contained in the supplementary information of the medical image. The medical image is assigned to the image generation apparatus 2a or 2b loaded with the recording medium having the corresponding density, based on the determined density, and is transmitted.

In the image recording apparatuses 2a and 2b, the density information of the recording medium loaded in the tray is sent to the Job Manager in advance by the transmission section (not illustrated). The Job Manager 4 receives this density information and stores the density information of the recording medium loaded in each of the image recording apparatuses 2a and 2b.

The image recording apparatuses 2a and 2b, having received the medical image, acquire the information on the size of the recording medium from the supplementary information of the medical image, select the tray corresponding to the size of the acquired recording medium size and sends the recording medium to the image recording section 29. At the same time, they selects the write pitch corresponding to the type of the medical image and record the medical image.

According to this arrangement, the Job Manager 4 determines the density of the recording medium, based on the supplementary information of the medical image and assigns the medical image to the image recording apparatuses 2a and 2b. This allows the medical image system 200 to provide comprehensive administration of the density of the recording medium. For example, even if there is a change in the specification of the medical image system, approximate action can be taken quickly in response to the change. Thus, effective system configuration is ensured.

It should be noted that the type of the recording medium densities is not restricted to the aforementioned description. For example, in the case of a mammographic image recording medium, Dmax = 3.6 through 4.0 is satisfactory. Dmax = 3.0 is satisfactory for the general medical image. The size of the recording medium may include a 14 x 14-inch size, 14 x 17-inch life size, 14 x 14-inch life size, 11 x 14-inch life size and 8 x 10-inch reduced size, in addition to the aforementioned a 14 x 17-inch size, 8 x 10-inch size and 11 x 14-inch size. It is also possible to arrange such a configuration that trays are provided in the number corresponding to the number of densities or sizes of the recording media, and the type of the write pitches that can be switched is set in conformity to the density of the recording medium.

The greater number of the write pitches that can be switched permits reproduction of a more preferred medical image in conformity to the particular type of the image generation apparatuses 1a through 1e. In the present embodiment, three or more write pitches can be set, although two write pitches have been described above. Alternatively, since the greater number of the write pitches that can be switched means less processing efficiency, it is also possible to provide two modes; one mode wherein the medical image is recorded by switching the write pitch in response to the type of the medical image pitch, and the other mode wherein recording the medical image at one write pitch for all medical images. According to the user requirements, these modes are switched to record the medical image.

It is also possible to make such arrangements that the image recording section 29 records the medical image based on the image signal outputted from the image processing section 23, and the write pitch is recorded. This arrangement allows the engineer to quickly find out the medical image that has been recorded at a wrong pitch, and permits the doctor to use the medical image as a reference at the time of diagnosis.

The detailed configurations and operations of the components of the medical image systems 100 and 200 in the present embodiment can be modified in an appropriate manner, without departing from the spirit of the present invention.

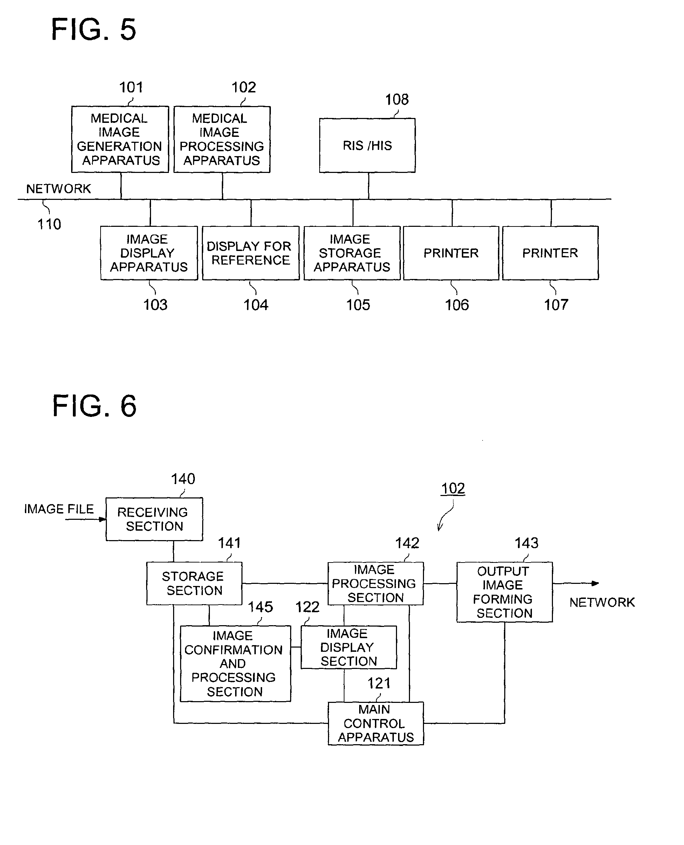

Referring to the drawing, the following describes the medical image system as another embodiment of the present invention:
The network system for medical use in Fig. 5 comprises:
   a medical image generation apparatus 101 for generating the medical image by the photographic modality of the CR (computed radiography) that causes the light to be emitted by using the excitation light to scan the stimulable phosphor panel with the radiographic image information of a subject (patient) recorded thereon, and for getting image information through photoelectric conversion of that light;
   a medical image processing apparatus 102 for outputting the image information after applying image processing by inputting an image file from the image generation apparatus 101; and
   an image display apparatus (viewer) 103, comprising a personal computer and a workstation, used by the doctor to carry out diagnosis by reference to the image.
The medical network system shown in Fig. 5 consists of:
   1) a reference terminal 104, further comprising a personal computer with a screen for display and a workstation, having inferior quality in resolution and others relative to the image display apparatus 103, because it refers to the image without carrying out diagnosis;
   2) an image storage apparatus (image server) 105, containing a personal computer and a workstation, used to store an image file in the image database, and to seek and read and reading an image from the image display apparatus 103 and reference terminal 104; and
   3) a plurality of printers 106 and 107 for outputting the image data from the medical image generation apparatus 101 or medical image processing apparatus 102, in the form of a visible image on the film made of a photosensitive material. The medical network system shown in Fig. 5 is linked with a RIS/HIS (order information management) 108 for the management of order information.
The medical network system is linked with various apparatuses 101 to 108 through the network 110 in such a way that information can be sent and received.

The following describes the details of the items A through H of the medical image processing apparatus 102 shown in Fig. 5:
A. Apparatus configuration
B. Information
C. File
D. Main information input and display
E. Image confirmation procedure
F. Output
G. Formation of output image
H. Utility function

### A. Apparatus configuration

Fig. 6 is a block diagram representing the configuration of the medical image processing apparatus 102.

a. The medical image processing apparatus 102 shown in Fig. 6 includes:
a main control apparatus 121 for controlling the entire image radiographing system; and
an image display section 122, containing a CRT display and liquid crystal display, for displaying the digital image monitor obtained by the medical image generation apparatus 101 or others. It can be composed of a personal computer and contains an information input apparatus such as an input keyboard and mouse.

As shown in Fig. 6, the medical image processing apparatus 102 further consists of:
a receiving section 140 for receiving an image file from the medical image generation apparatus 101;
a storage section 141, composed of a hard disk apparatus and RAM, for storing the information of the received image file and image information after image processing;
an image processing section 142 for processing of the image information in the image file;
an output image forming section 143 for forming the output image to be outputted to an external apparatus; and
an image confirmation and processing section 145 for display a thinned out image on the image display section 122 to confirm the received image. The main control apparatus 121 controls the receiving section 140, output image forming section 143, image confirmation and processing section 145 and image display section 122.

b. The following describes the functions of the medical image processing apparatus 102: The functions are controlled by the main control apparatus 121.
<1> An image file is received by the receiving section 140 through the medical image generation apparatus 101.
<2> The image file is temporarily stored by the storage section 141.
<3> The image quality is checked using the reduced image created by the image confirmation and processing section 145.
<4> Image processing is applied by the image processing section 142.
<5> An output image is formed by the output image forming section 143.
<6> The image is outputted to the image display apparatus 103, image storage apparatus 105 and printers 106 and 107 a through the memory device 110.

### B. Information

The information handled by the medical image processing apparatus 102 can be classified into the following five categories:

### a. Condition information

The condition information receives an image file and outputs it to an external apparatus such as an image server 105 as a processed image file. It contains the following:

### (a) Image processing information

This is the information on the processing of gradation and frequency in the image processing section 142.

### (b) Output apparatus information

This is the information on the external apparatus such as an image server 105 for reproducing and outputting the image data. It specifies the output range, scaling factor, output format (multi-format, split photographic format), overlay, and presence/absence of gradation processing and frequency processing for each output apparatus such as an image server 105.

### (c) Overlay information

This is the information on the presence/absence and position of an overlay such as AP/PA R/L comment and deletion mark (double line, "x" mark, etc.).

The position of the overlay on the screen can be changed as desired.

When the AP and PA of the lead marker have been incorrectly radiographed, a deletion mark is overwritten on the lead marker portion of the screen, and the AP/PA and other letters are overlaid at a desired position, whereby an error is completely corrected.

### (d) Special designation

Protect information: After the image has been outputted, the image file is stored until it is unprotected.

Pending information: This information suspend transfer of the image data. This information is designated when the image is to be transferred after it has been reviewed later.

Priority information (emergency information): This is designated when urgent outputting is needed in the case of emergency radiographing.

### b. Patient information

Information on a patient

### (a) Patient ID information

Includes a patient ID number, name, gender, and date of birth.

### (b) Order information

This is information on the order placed by a doctor for radiographing, and contains information on the time and date on the request for examination, as well as instruction on the method to be used.

### c. Implementation information

Information on the result of data reception and processing.

### (a) Result of reception:

Includes information on the date and time of radiographing.

### (b) Result of image processing

A result of calculating the image processing parameter. Based on this result, image data is processed at the time of outputting.

### (c) System information

Includes information on the system configuration and part of the system information at the time of radiographing

### d. System information

(a) Information for management and control of the system shown in Fig. 5
(b) System configuration shown in Fig. 5 (name of the external apparatus connected to the search operation 5, and others)
(c) Parameter table for controlling the equipment constituting the system configuration shown in Fig. 5
(d) Setting information on the medical image generation apparatus 101 as an input apparatus
(e) Setting information on the outputting apparatus such as information on the printer 106 and HOST

### e. Image data

(a) Image received from the medical image generation apparatus 101
(b) Reduced image data for display created from the image data for image confirmation
(c) Reduced image data for processing of a reduced image for display by the image confirmation and processing section 145
(d) Output image data having been subjected to processing of gradation and frequency

### C. File

The file handled in the medical image processing apparatus 102 is stored in the storage section 141 and can be classified into the following seven categories:

### a. Condition file

The condition key is used to set the image processing conditions and outputting conditions for the image file in advance. A condition file is provided for each condition key. The condition file is formed of the aforementioned condition information. It is classified according to the part of an object to be radiographed (abdomen, lung field, head), patient position for radiographing (upright, lying position), direction for radiographing (front, side etc.), patient characteristics (gender, age, physical size), name of disease, name of the engineer in charge, etc. Corresponding names and information for radiographing are inputted in advance. The main control apparatus 121 sets a condition file group for each of multiple categories classified, sets multiple condition files for the set condition file group, stores them in the storage section 141, and selects one condition best suited to image reception.

### b. Image header file

After reception, an image header file is created. The image header is made up of a reserved file for the radiographing (i.e. radiographing information, patent information) and radiographing implementation information. Reference is made to the image header file when a user wishes to make modification by reference to the radiographing information, patient information and radiographing implementation information.

### c. Reduced image file

An image data created by reducing the image data in fractions.

### (a) Reduced image data for display

This reduced image for display is used as the data displayed on the image display section 122 shown in Fig. 6.

### (b) Reduced data for image processing

Reduced data for calculating the parameter for image processing.

The reduction rate is determined in such a way that the one pixel subsequent to reduction will be equal to the length specified in advance. This arrangement allows the difference in the read pixel size to be corrected using the reduced image. The image processing parameter is calculated using the reduced image for image processing. The image data is not used.

### d. Image file

(a) The image file consists of the supplementary information (image header) of the image and image data.
(b) The image header contains condition information, patient information and implementation information.
Reference is made to the image header when a user wishes to make modification by reference to the condition information, patient information and implementation information.

### e. Output image file

This is the output image data where the specified processing, out of processing of frequency and gradation, overlay, rotation and enlargement/reduction, has been applied.

### f. System file

The system file is created by forming the aforementioned system information into a file.

### D. Input and display of main information

### a. Reception image information display

Provides thumbnail display of the reception image.

### b. Output information display

<1> The output size, direction, trimming position, output position and enlarge/reduce method are specified. They are registered in the condition file in advance.
<2> When the condition key is selected, the output region and output image region are determined under the predetermined condition, and are displayed on the screen of the image display section 122. The size of the output region display area is to be the maximum output region at the time of outputting. The output region and output image region are graphically displayed in the output region display area. This arrangement allows the output region and output image region to be selected and confirmed for each apparatus.

### c. Overlay information

<1> This information designates if the AP, PA, R, L, comment and graduation should be overlaid or not, and specifies the position of overlay if they are to be overlaid. The information is registered in the condition file in advance.
<2> The output image is displayed in the output region display area on the screen of the image display section 122, wherein overlay information is graphically represented.
<3> The appropriate overlay can be selected and the position can be specified.
<4> It is possible to verify that there is no any hidden and invisible portion in the overlay. If any trouble occurs to diagnosis due to overlay, the position is shifted.

### d. On-line information input/output from RIS

<1> Doctor's order is inputted. The inputted order is converted into the format of this system and is stored in a reservation file. The condition and method for radiographing are converted under the corresponding radiographing condition key.
<2> The image header file is converted into the RIS side format and is outputted.

### e. Image list

Image files can be displayed as a list.

### E. Image confirmation procedure

### a. Operation of the system at the time of image confirmation

(1) The image file is received from the medical image generation apparatus 101 and is stored in the storage section 141.
(2) The image file stored in the storage medium of the storage section 141 is reduced at a predetermined reduction rate by the image confirmation and processing section 145.
(3) The reduced image is displayed on the screen of the image display section 122 sequentially.
(4) Upon completion of reception and display, the digital image information is processed in a predetermined manner by the radiographing condition key, and is redisplayed on the image display section 122. The reduced image is used to determine the parameter for image processing.
(5) The images sequentially displayed on the image display section 122 and having been subjected to processing of gradation are displayed again.
(6) When the operator watches the reception image displayed on the December image display section 122, and determines that there is no problem, the key to confirm termination of reception is pressed from the character information input apparatus, and the image confirmation step terminates.
(7) If the patient information, image processing method and output method are to be changed, new information can be inputted from the character information input apparatus.
(8) When the image confirmation key has been pressed, the confirmation of the present image terminates, and the next screen is displayed automatically.
(9) If there is any problem with the image, image processing can be changed. Detailed modification of image processing can be made later, as a reservation function.
(10) When the image confirmation key has been pressed, the image confirmation terminates. Then the following processing is performed:
   <1> The image file is stored in the storage section 141 as a confirmed image file.
   <2> The image having undergone the step of terminated the image confirmation is registered in the queue for outputting to the external apparatus.
   <3> Then the received image file is displayed and the image confirmation is enabled.
(11) When the reservation key has been pressed, image confirmation terminates.

### F. Output

<1> Outputting is performed asynchronously with reception and image confirmation.
<2> The queue is created for each external apparatus and is placed under management. Each queue acts independently of the other without affecting it. Thus, outputting is carried out asynchronously for each apparatus.
<3> The name of the external apparatus in whose queue the image is registered is stored in the storage section 141 in the form of a queue registration table. It is updated for every registration to the queue and for every deletion, and is placed under management.
<4> The images registered in the queue are outputted to the external apparatus in the order of being registered. The images having been outputting are deleted from the queue.
<5> When outputting is to be implemented, the image file stored in the storage section 141 is specified using the number registered in the queue.
<6> The output image is formed under the conditions stored in the image file.

### G. Formation of output image

a. The output image is formed mainly by the following processing:
   <1> The image data is read from the storage section 141 into the image memory.
   <2> Frequency processing is applied.
   <3> Equalization processing is applied.
   <4> Gradation processing is applied.
   <5> The image is rotated.
   <6> Mirroring is performed.
   <7> Enlargement and reduction are performed.
   <8> Overlay is performed.
b. Whether steps <2> through <8> are to be applied or not can be specified for each output apparatus, using the condition information.
c. Designation can be given in such a way that the image data having been subjected to specified processing of <2> through <8> is stored in the form of a processed image data file. This step eliminates repetition of the common processing of the image to be outputted to each outputting apparatus.
d. When there is a difference in the scaling factor of the image to be outputted to each outputting device, the processed image is stored up to the step <6>. Then when it is transferred to another apparatus, the image having been processed up to step <6> is read out. Only the processing of <7> and <8> is performed, and the image is transferred, whereby the time required for steps <2> through <6> can be saved.
e. Steps <5> and <6> are executed simultaneously with any one of steps <2>, <3> and <4>. This will reduce the number of accesses to the memory, with the result that processing time is cut down.

### H. Utility function

a. There are several functions as a utility for the user. Utility function is restricted for each general user, manager and manufacturer by the password. Modification of the information on image in particular requires manager's password to ensure security.

### b. Image file operation

<1> The image file is displayed and the information on the stored image is displayed on the image display section 122 in the order of reception.
<2> When a desired image is selected from the list, the patient information, condition information and image are displayed in the same form as that on the screen at the time of image confirmation.
<3> The patient information, image processing method and outputting method can be changed.
<4> The image for which "Pending" has been specified at the time of radiographing is released from the status of "pending" by reconfirmation carried out here.
<5> Whether the image is to be outputted to each external apparatus or not can be changed. The order of outputted can also be changed.

### c. Radiographing record and irradiation record

<1> The radiographing information and Statistics processing is applied to patient information, and the radiographing record and irradiation record are supplied to the user.
<2> A list can be outputted, indicating the number of shots for each radiographed region of an object during the designated period of time and the radiographing conditions.

### d. Customization

The screen and maneuverability can be customized for each user.

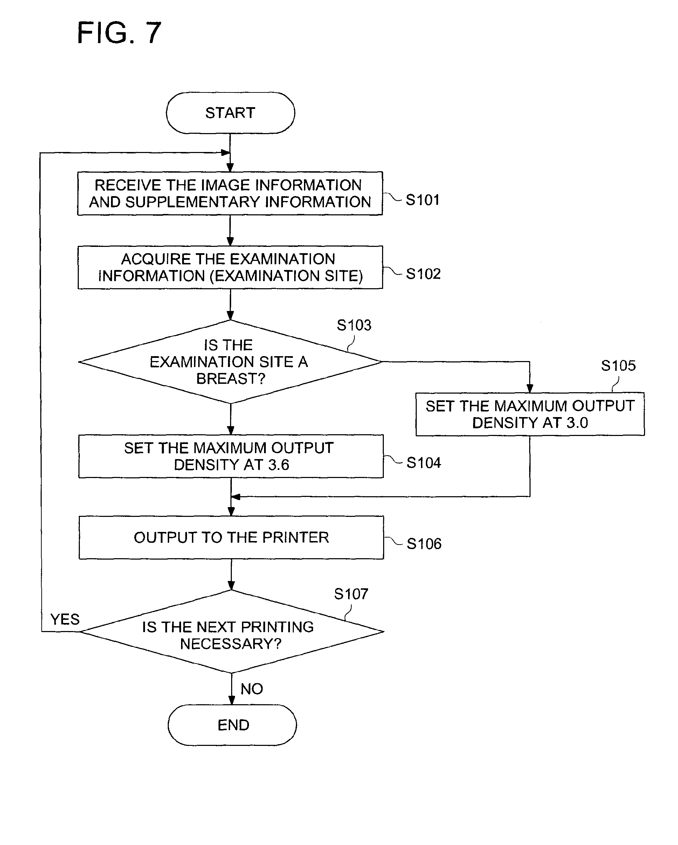

In the image recording apparatus 2 shown in Figs. 5 and 6, the following describes the first operation wherein the maximum density setting (density range) of the output film is changed according to the examination information of the subject (information on examination part of an object), with reference to the flowchart shown in Fig. 7.

The medical image processing apparatus 102 shown in Figs. 5 and 6 receive the image information and image supplementary information including the patient and examination information of that image from the medical image generation apparatus 101 (Step S101). Predetermined image processing is applied to the image information as described above, on the one hand, and acquires information on the examination part of an object (e.g. information on the part of an object at the time of radiographing), out of the examination information of the patient from the supplementary information, on the other (Step S102).

Then the evaluation is made to determine whether the part of an object examined is the breast (mammography) or not (Step S103). If it is the breast, the setting of the maximum output density is changed to 3.6 (Step S104). Further, if the examined part of an object is not a breast but a head, for example, then the setting of the maximum output density is changed to 3.0 (Step S105). This is followed by the step of outputted the processed image information to the printers 106 and 107 (Step S106). When the next printing is carried out (Step S107), the system goes back to the Step S101.

The main control apparatus 121 rewrites the lookup table (LUT) for conversion between the pixel value and density in the range of the density so that maximum output density will be changed. This rewritten LUT is put into the supplementary information and is outputted to the printer together with the image information, whereby the aforementioned change of the setting of the machining output density is implemented. For example, in the case of a laser imager where the visible image is developed after the printer has formed a latent image on a film by laser beams, the maximum density can be changed by adjusting the amount of laser beam at the time of exposure of the film.

As described above, in the case of the breast image obtained by mammography where film density higher than the normal level is suitable for diagnosis, the maximum output film density can be automatically set to the maximum level (3.6) which is higher than that of other part of an object, depending on the examination information of the patient and information on the examination part of an object. Further, when the examination part is a head, the maximum output film density can be set to 3.0 and can be outputted. As described above, automatic switching of the setting of the maximum output film density reduces user operation error.

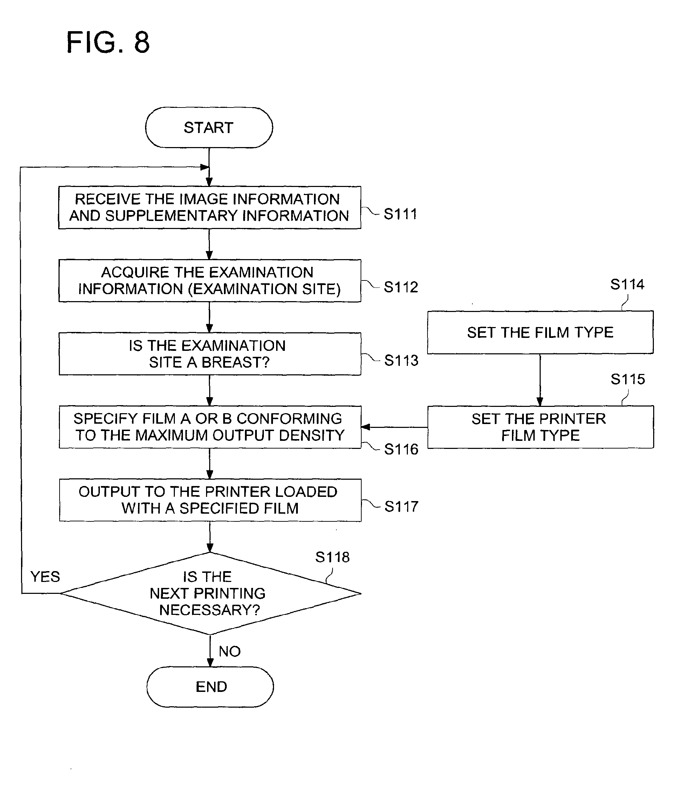

With reference to the flowchart shown in Fig. 8, the following describes the second operation wherein outputting is performed by specifying a printer in conformity to the image with maximum density (density range) setting changed in the medical image processing apparatus 102 shown in Figs. 5 and 6.

The medical image processing apparatus 102 shown in Figs. 5 and 6 receives the image supplementary information including the patient/examination information from the medical image generation apparatus 101 (Step S111). Predetermined image processing is applied to image information, and the examination part information (e.g. information of the part at the time of radiographing) of the patient examination information is obtained from the supplementary information (Step S112).

The maximum output film density suitable for the image is set according to the examination part information (Step S113). Similarly to the case Fig. 7, this setting can be made by determining whether the examination part of an object is a breast or not, for example.

The type of the film corresponding to the maximum output density is set (Step S114), and the type of the film in the printers 106 and 107 in Fig. 5 is set (Step S115). For example, the film A corresponding to the maximum output density of 3.6 suitable for the case of the examination part of an object being the breast is set. This film A being loaded on the printer 106 is also set and is stored in the storage section 141 shown in Fig. 6. Further, the film B corresponding to the maximum output density of 3.0 is set. At the same time, this film B being loaded on the printer 107 is also set and is stored in the storage section 141 shown in Fig. 6.

The film A or B corresponding to the maximum output film density having been set as described above is specified (Step S116), and the image information is outputted to the printer 106 or 107 where the specified film is set (Step S117). When the next printing is performed (Step S118), the system returns to Step S111.

As described above, the setting of the maximum film density can be changed according to at least one of the obtained examination information and examination part of an object information of the patient, and the image can be outputted to the film specified according to this maximum output film density. To put it another way, it can be outputted to the printer 106 or 107 loaded with the film A or B suitable for the image where the setting of the maximum film density has been changed. This arrangement provides an image suitable for the diagnosis of a subject. For example, the image where mammographic examination part is the breast can be outputted to the film A on the printer 106 corresponding to the maximum density of 3.6. Further, the image where the examination part is a head can be outputted to the film B on the printer 107 corresponding to the maximum density of 3.0. As described above, automatic switching of the setting of the maximum film density reduces user operation error.

In the aforementioned Step S117, for example, when one printer 106 has two film trays for accommodating films A and B, the film tray loaded with the film A or B suitable for the image where the setting of the maximum film density is changed is selected and the image is outputted, thereby getting the image suitable for the diagnosis of the subject, in the similar manner.

As described above, automatic selection is made of the printer or film tray specified in conformity to the image where the setting of the maximum film density is changed by synchronization between the film type (A, B) of the printer and the maximum output film density set on the medical image processing apparatus, and the image can be outputted to the film corresponding to that maximum film density.

In Fig. 8, information on the film type of the printers 106 and 107 is set in the medical image processing apparatus in advance. It is also possible to make such arrangements that it is obtained from each of the printers 106 and 107. If there is any setting (development temperature, development speed and output format) other than the maximum density setting, depending on the examination part of an object, it is possible to use the same handling procedure as that in the maximum density setting on each output apparatus and medical image processing apparatus.

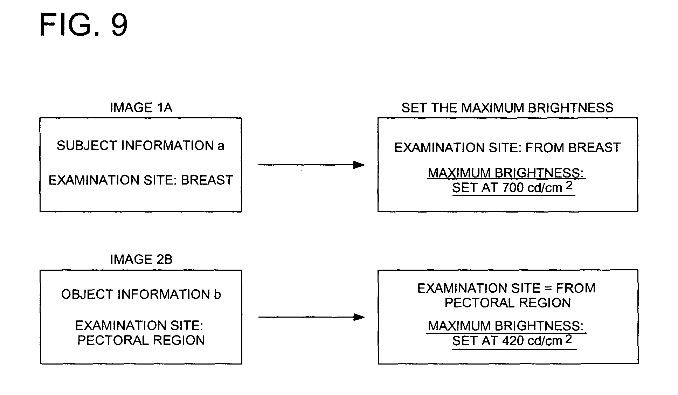

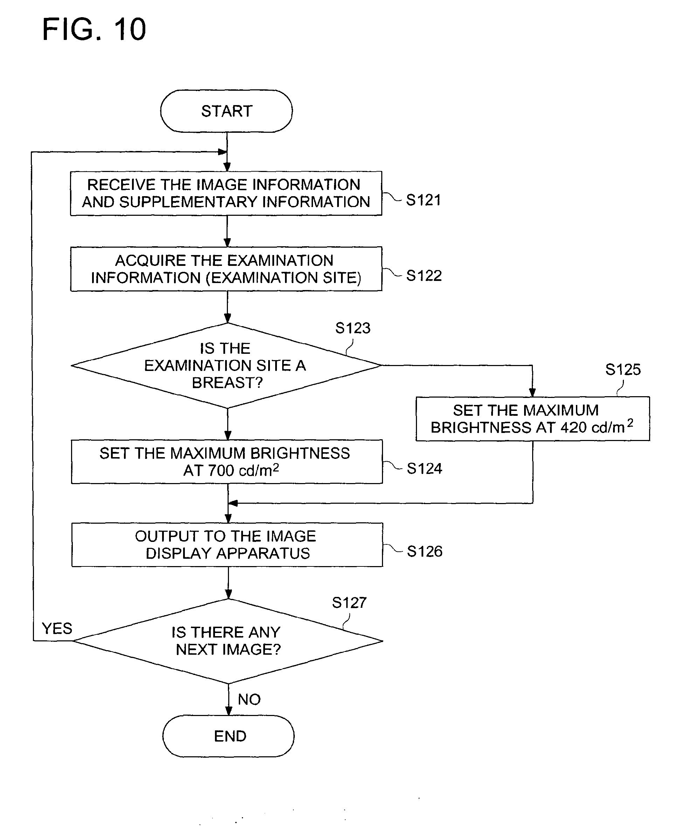

With reference to Figs. 9 and 10, the following describes the structure of the medical image processing apparatus 102 shown in Figs. 5 and 6, where the setting of the maximum brightness of the display (monitor) of the image display apparatus 103 shown in Fig. 5 is changed according to the information on the subject.

Fig. 9 schematically represents the setting of the maximum brightness of the monitor with respect to the examination part of the image, in the medical image processing apparatus 102 shown in Figs. 5 and 6. Fig. 10 is a flowchart representing the operation of changing the setting of the maximum brightness of the monitor based on the subject information, in the medical image processing apparatus 102 shown in Figs. 5 and 6.

In the medical image processing apparatus 102 shown in Figs. 5 and 6, it is possible to change the maximum brightness setting of the monitor when the diagnostic image is displayed on the image display apparatus 103, based on the subject information (e.g. part information and specific condition key at the time of radiographing).

As described above, when an image is outputted to the film by an outputting device such as an imager, the maximum density can be changed on the imager side by adjusting the amount of laser light. In the case of a liquid crystal display or CRT monitor, the maximum density is fixed at the minimum brightness, so this adjustment is made by expanding the entire dynamic range by changing the brightness and the minimum density (maximum brightness).

For example, when the examination part of the subject information b of image 1A is a breast as shown in Fig. 5, the setting of the maximum brightness of the monitor is changed to 700 cd/m². Further, if the examination part of the object information b of image 2B is a head, the setting of the maximum brightness of the monitor is changed to 420 cd/m².

The maximum brightness setting can be obtained by changing the JND range (Just-Noticeable Difference, the minimum brightness difference perceptible to human sense) - the maximum brightness on the monitor -, for example, in response to the maximum density of the mammographic image set at 3.6 in the case of a film and the maximum density of the image for other parts set at 3.0.
(1) JND range is 876 for the density of 3.6, and 781 for the density of 3.0, assuming that the minimum density is 0.2, viewing box brightness 3000 cd/m² and reflection brightness (external light such as reflected light of a fluorescent lamp) 0 cd/m².
(2) JIS range for the maximum brightness (700 cd/m²) corresponding to the density of 3.6 is 698, assuming that the maximum brightness of the monitor is 700 cd/m² and the minimum brightness 0.7 cd/m².
(3) The JND range at other parts is 420 d/m², assuming that the JND range has the same ratio as that when the mammographic image density is 3.6 and image density of other parts 3.0.

Thus, the maximum brightness is set at 700 cd/m² for the mammographic image and 420 cd/m² for the image of other parts.

In the case of other parts, however, the user can determine the set value by taking glare and other factors into account, if the brightness is 420 cd/m² or more. For example, it is possible to select 600 cd/m².

In the medical image processing apparatus 102 shown in Figs. 5 and 6, the following describes the operation of display the image by changing the maximum brightness setting of the monitor is determined, depending on the examination information of the subject (examination part information). As shown in Fig. 10, the medical image processing apparatus 102 shown in Figs. 5 and 6 receives the image information and the image supplementary information including the patient/examination information of the image from the medical image generation apparatus 101 (Step S121). As described above, predetermined processing is applied to the image information, and the examination part information (e.g. part information at the time of radiographing) out of the patient examination information is acquired from the supplementary information (Step S122).

Then the evaluation is made to determine whether the part examined is the breast (mammography) or not (Step S123). If it is the breast, the maximum brightness of the monitor of the image display apparatus 103 shown in Fig. 5 is set at 700 cd/m² (Step S124). Further, if the examined part is not a breast but a pectoral region, for example, then the maximum brightness of the monitor is set at 420 cd/cm² (Step S125).

Then the image information having been subjected to image processing is outputted to the image display apparatus 103 shown in Fig. 5 (Step S126). If there is the next screen (Step S127), the system goes back to Step S121.

The maximum brightness in the aforementioned Step S124 or 125 is set by putting the maximum brightness information into the supplementary information and by sending it to the image display apparatus 103 of Fig. 5 as an image file. To put it another way, the command for setting the maximum brightness and the LookUp Table (LUT) for conversion between the pixel value and brightness are sent together with the image information.

As described above, according to Figs. 9 and 10, the further optimized image display can be ensured for diagnosis corresponding to the examination part, by setting the maximum brightness of the display apparatus at the output destination, based on the examination part of the object. For example, if the image where mammographic examination part is the breast in mammography is to be displayed on the display monitor, the image suitable for the breast can be displayed and observed, by setting the maximum brightness to a higher level and by expanding the dynamic range. Further, this arrangement reduces the user fatigue and allows him or her to observe the diagnostic image by ensuring the feel of making an effective use. Further, this arrangement provides the information on the subject and permits automatic selection of the maximum brightness setting, thereby minimizing both the user operation and operation errors.

To reduce the eye fatigue and glare at the time of image diagnosis, the display monitor has been often used with maximum brightness reduced, according to the prior art. Further, there is a difference in the required dynamic range, depending on the part of the subject. The diagnosis of breasts requires a wider dynamic range than that of other parts. However, since the monitor brightness is reduced, hardware capability has not been utilized, in the prior art. In the present embodiment, by contrast, when the mammographic image is displayed, the hardware capability is fully utilized by using the dynamic range widened by setting the maximum brightness at a higher level. This method ensures easy observation of a mammographic image at the time of image diagnosis.

With reference to Fig. 10, description has been made of the case of changing the setting of the maximum brightness of the monitor of the image display apparatus 103 when the diagnostic image is observed by a doctor. The setting of the maximum brightness can be similarly changed on the image display section 122 of the medical image processing apparatus 102 (Fig. 6) and the monitors attached to the reference terminal 104 or the image storage apparatus 105. These display monitors can be liquid crystal displays or CRTs.

The operations of the medical image processing apparatus 102 in the present embodiment are controlled by the main control apparatus 121 of Fig. 6, according to the program stored in the storage section 141, and required information processing is applied.

The present invention has been described with reference to preferred embodiments. It should be noted, however, that the present invention is not restricted thereto. Various modifications are possible without departing from the engineering spirit of the present invention. It goes without saying that the medical image generation apparatus includes, in addition to the CR apparatus where radiographic image is read from the stimulable phosphor panel with the radiographic image information of a patent recorded thereon, a radiographic imaging apparatus using an X-flat panel detector, a CR apparatus (computed radiography), a MRI apparatus (magnetic resonance imaging apparatus), a DR apparatus (digital radiography), a US apparatus (ultrasound) and others. Further, these medical image generation apparatuses can be linked to the network system according to the present invention.

### [EFFECTS OF THE INVENTION]

The medical image generation apparatus, medical network system and the program for the medical image processing apparatus of the present invention provides an image suitable for diagnosis of the examination part of a subject by changing the setting of the output density range and setting of the maximum brightness for display, based on the examination information, when image processing is applied to the received medical image information and the result is sent to other apparatuses.

## Claims

1. A medical image system comprising:
an image data input section for inputting image data including medical image data of a radiographed object and supplementary information supplemented with the medical image data;
a plurality of outputting sections for outputting a medical image on the basis of the medical image data;
a setting section for setting at least one of a setting density range and a setting brightness range of the medical image to be outputted on the basis of the inputted supplementary information;
an information obtaining section for obtaining at least one of density information including a output density range and brightness information including a output brightness range which each of the outputting sections can output, from each of the outputting sections;
a selecting section for selecting at least one of the outputting sections which can output the medical image data by comparing the setting density range with each of the output density range or comparing the setting brightness range with the output brightness range; and
a transmitting section for transmitting the medical image data with the supplementary information to the selected outputting sections.

2. The medical image system of claim 1, wherein the outputting section comprises an image recording section for recording a medical image on the basis of the medical image data on a recording medium,
wherein the setting section sets the setting density range,
wherein the information obtaining section obtains the density information including the output density range which the image recording section can record,
wherein the selecting section selects at least one of the image recording sections by comparing the setting density range with each of the output density range, and
wherein the transmitting section transmits the medical image data with the supplementary information to the selected image recording sections.

3. The medical image system of claim 1, wherein the outputting section comprises a displaying section for displaying the medical image,
wherein the setting section sets the setting brightness range,
wherein the information obtaining section obtains the brightness information including the output brightness range which the displaying section can display,
wherein the selecting section selects at least one of the displaying sections which can display the medical image by comparing the setting brightness range with each of the output brightness range, and
wherein the transmitting section transmits the medical image data with the supplementary information to the selected displaying sections.

4. The medical image system of claim 1, wherein the supplementary information includes examination information related to the radiographed object, and
wherein the setting section sets at least one of a setting density range and a setting brightness range on the basis of examination information.

5. The medical image system of claim 1, wherein the supplementary information includes part information of a part of an object to be radiographed, and
wherein the setting section sets at least one of a setting density range and a setting brightness range on the basis of part information.

6. The medical image system of claim 1, wherein the supplementary information includes a recording pixel size corresponding with a read pitch at the time of obtaining the medical image data, and
wherein the setting section sets at least one of a setting density range and a setting brightness range on the basis of the recording pixel size.

7. The medical image system of claim 1, wherein the supplementary information includes maximum density information, and
wherein the setting section sets at least one of a setting density range and a setting brightness range on the basis of maximum density information.

8. The medical image system of claim 2, wherein the image recording section comprises a switching device for switching the write pitch at the time of the recording on the basis of supplementary information.

9. A medical image system comprising:
an image data input section for inputting image data including medical image data of a radiographed object and supplementary information supplemented with the medical image data;
a outputting section for outputting a medical image on a plurality of recording mediums different in density, on the basis of the medical image data;
a setting section for setting a setting density range on the basis of the inputted supplementary information;
an information obtaining section for obtaining at least one of density information including a output density range which can be outputted, from each of the recording mediums;
a selecting section for selecting at least one of the image recording mediums on which can be outputted the medical image data by comparing the setting density range with each of the output density range; and
a controlling section for controlling the outputting section so as to output the medical image on the selected image recording medium.

10. The medical image system of claim 9, wherein the supplementary information includes examination information related to the radiographed object, and
wherein the setting section sets the setting density range on the basis of examination information.

11. The medical image system of claim 9, wherein the supplementary information includes part information of a part of an object to be radiographed, and
wherein the setting section sets the setting density range on the basis of part information.

12. The medical image system of claim 9, wherein the supplementary information includes a recording pixel size corresponding with a read pitch at the time of obtaining the medical image data, and
wherein the setting section sets the setting density range on the basis of the recording pixel size.

13. The medical image system of claim 9, wherein the supplementary information includes maximum density information, and
wherein the setting section sets the setting density range on the basis of maximum density information.

14. The medical image system of claim 9, wherein the outputting section comprises a switching device for switching the write pitch at the time of the recording on the basis of supplementary information.

15. The medical image system of claim 9, wherein the outputting section has the plurality of trays in each of which load the recording mediums according to the density of each of the recording mediums.

16. A medical image processing method comprising the steps of:
inputting image data including medical image data of a radiographed object and supplementary information supplemented with the medical image data;
outputting a medical image on the basis of the medical image data from a plurality of outputting sections;
setting at least one of a setting density range and a setting brightness range of the medical image to be outputted on the basis of the inputted supplementary information;
obtaining at least one of density information including a output density range and brightness information including a output brightness range which each of the outputting sections can output, from each of the outputting sections;
selecting at least one of the outputting sections which can output the medical image data by comparing the setting density range with each of the output density range or comparing the setting brightness range with the output brightness range; and
transmitting the medical image data with the supplementary information to the selected outputting sections.

17. The medical image processing method of claim 16,
wherein the outputting section comprises an image recording section for recording a medical image on the basis of the medical image data on a recording medium,
wherein the setting step sets the setting density range,
wherein the obtaining step obtains the density information including the output density range which the image recording section can record,
wherein the selecting step selects at least one of the image recording sections by comparing the setting density range with each of the output density range, and
wherein the transmitting step transmits the medical image data with the supplementary information to the selected image recording sections.

18. The medical image processing method of claim 16,
wherein the outputting section comprises a displaying section for displaying the medical image,
wherein the setting step sets the setting brightness range,
wherein the obtaining step obtains the brightness information including the output brightness range which the displaying section can display,
wherein the selecting step selects at least one of the displaying sections which can display the medical image by comparing the setting brightness range with each of the output brightness range, and
wherein the transmitting step transmits the medical image data with the supplementary information to the selected displaying sections.

19. The medical image processing method of claim 16,
wherein the supplementary information includes examination information related to the radiographed object, and
wherein the setting step sets at least one of a setting density range and a setting brightness range on the basis of examination information.

20. The medical image processing method of claim 16,
wherein the supplementary information includes part information of a part of an object to be radiographed, and
wherein the setting step sets at least one of a setting density range and a setting brightness range on the basis of part information.

21. The medical image processing method of claim 16,
wherein the supplementary information includes a recording pixel size corresponding with a read pitch at the time of obtaining the medical image data, and
wherein the setting step sets at least one of a setting density range and a setting brightness range on the basis of the recording pixel size.

22. The medical image processing method of claim 16,
wherein the supplementary information includes maximum density information, and
wherein the setting step sets at least one of a setting density range and a setting brightness range on the basis of maximum density information.

23. The medical image processing method of claim 17,
wherein the image recording section comprises a switching device for switching the write pitch at the time of the recording on the basis of supplementary information.

24. A medical image processing method comprising the steps of:
inputting image data including medical image data of a radiographed object and supplementary information supplemented with the medical image data;
outputting a medical image on a plurality of recording mediums different in density, on the basis of the medical image data, from a outputting section;
setting a setting density range on the basis of the inputted supplementary information;
obtaining at least one of density information including a output density range which can be outputted, from each of the recording mediums;
selecting at least one of the image recording mediums on which can be outputted the medical image data by comparing the setting density range with each of the output density range; and
controlling the outputting section so as to output the medical image on the selected image recording medium.

25. The medical image processing method of claim 24,
wherein the supplementary information includes examination information related to the radiographed object, and
wherein the setting step sets the setting density range on the basis of examination information.

26. The medical image processing method of claim 24,
wherein the supplementary information includes part information of a part of an object to be radiographed, and
wherein the setting step sets the setting density range on the basis of part information.

27. The medical image processing method of claim 24,
wherein the supplementary information includes a recording pixel size corresponding with a read pitch at the time of obtaining the medical image data, and
wherein the setting step sets the setting density range on the basis of the recording pixel size.

28. The medical image processing method of claim 24,
wherein the supplementary information includes maximum density information, and
wherein the setting step sets the setting density range on the basis of maximum density information.

29. The medical image processing method of claim 24,
wherein the outputting section comprises a switching device for switching the write pitch at the time of the recording on the basis of supplementary information.

30. The medical image processing method of claim 24,
wherein the outputting section has the plurality of trays in each of which load the recording mediums according to the density of each of the recording mediums.
